# EUROPÄISCHE PATENTSCHRIFT

(11) **EP 1 487 069 B1**
(45) Veröffentlichungstag und Bekanntmachung des Hinweises auf die Patenterteilung: **16.11.2005**
(21) Anmeldenummer: 03013096.7
(22) Anmeldetag: 11.06.2003
(51) Int. Cl.: H01S 3/00, A61B 18/20, A61F 9/008

(54) **Sicherheitsvorrichtung für eine Laseranordnung**
Safety device for a laser system
Dispositif de sécurité pour un système laser

(43) Veröffentlichungstag der Anmeldung: 15.12.2004
(73) Patentinhaber: TRUMPF Laser GmbH + Co. KG, 78713 Schramberg (DE)
(72) Erfinder: Elfinger, Otto, 78132 Hornberg (DE)
(74) Vertreter: Kohler Schmid Möbus

(56) Entgegenhaltungen:
- EP-A- 1 044 755
- EP-A- 1 265 322
- DE-A- 3 541 965
- DE-A- 4 127 421
- DE-A- 4 204 721
- US-B1- 6 285 693

## Beschreibung

Die vorliegende Erfindung betrifft eine Sicherheitsvorrichtung für eine Laseranordnung, mit einem drehbar gelagerten optischen Element, das in seiner Grundstellung im Strahlengang eines Laserstrahls angeordnet ist, und mit einem Stellantrieb, der das optische Element aus der Grundstellung gegen die Wirkung einer Rückstellfeder in eine Betriebsstellung dreht, wobei die Rückstellfeder am optischen Element oder an dessen Halter exzentrisch zur Drehachse befestigt ist.

Eine solche Sicherheitsvorrichtung ist beispielsweise durch die EP-A-1 044 755 bekannt geworden.

Sicherheitsvorrichtungen für Laseranordnungen sind bekannt und dienen bei motorbetriebenen Spiegeln dazu, bei einem Defekt der Motorsteuerung keine gefährliche Situation entstehen zu lassen. Dazu ist eine als Schraubenfeder ausgestaltete Rückstellfeder vorgesehen, die im Fehlerfall den Spiegel in eine sichere Position zieht. In dieser sicheren Position wird der Laserstrahl vom Spiegel in einen Absorber umgelenkt, in dem die Lichtenergie in Wärme umgewandelt wird. Um die Rückstellfeder zu testen, wird bei jeder Initialisierung (Neustart) der Laseranordnung die Rückstellfeder gespannt und dann die Motoransteuerung abgeschaltet. Nun muss die Rückstellfeder den Spiegel in die sichere Position ziehen, die über einen Schalter erkannt wird. Ist die Rückstellfeder gebrochen, wird die sichere Position nicht erreicht und somit ein Bruch der Rückstellfeder erkannt. Allerdings kann bei diesen bekannten Sicherheitsvorrichtungen ein Bruch der Rückstellfeder nur beim Neustart, nicht aber während des Betriebs erkannt werden.

Bei der aus EP-A-1 044 755 bekannten Sicherheitsvorrichtung für eine Laseranordnung ist ein Shutter zwischen einer den Strahlengang des Laserstrahls freigebenden Stellung und einer den Strahlengang unterbrechenden Stellung drehbar gelagert. Der Shutter ist durch eine Feder in die unterbrechende Stellung vorgespannt und wird mittels einer Antriebsspule in die freigebende Stellung bewegt.

Weiterhin ist aus der DE 42 04 721 A eine Sicherheitsvorrichtung für eine Laseranordnung bekannt, die einen Tauchspulenshutter mit einer Schließblende aufweist. Die Schließblende ist in den Strahlengang des Laserstrahls durch eine Zugfeder vorgespannt, welche als Induktivität eines Parallelresonanzkreisoszillators benutzt wird. Ein Bruch der Zugfeder wirkt sich als Frequenzänderung des Parallelresonanzkreisoszillators aus, die von einem Rechner detektiert wird.

Aufgabe der vorliegenden Erfindung ist es daher, eine Sicherheitsvorrichtung der eingangs genannten Art dahingehend weiterzubilden, dass ein Bruch der Rückstellfeder auf einfache Weise auch während des Betriebs der Laseranordnung festgestellt werden kann.

Diese Aufgabe wird erfindungsgemäß dadurch gelöst, dass die Rückstellfeder im Stromkreis einer elektrischen Überwachungsschaltung angeordnet ist, welche eine Unterbrechung des über die Rückstellfeder fließenden Stroms anzeigt.

Im Fall eines Bruchs der Rückstellfeder ist der Stromfluss über die Rückstellfeder unterbrochen, was von der Überwachungsschaltung sofort erkannt und angezeigt wird. Dadurch werden mögliche Schäden durch einen Zweitfehler (z.B. Zerstörung des Motortreibers nach einem Bruch der Rückstellfeder) und Schäden durch das Laserlicht, welches durch eine gebrochene Rückstellfeder abgelenkt wird, vermieden. Bei der erfindungsgemäßen Sicherheitsvorrichtung entfällt der beim Stand der Technik erforderliche Testlauf der Rückstellfeder, wodurch bei der Initialisierungsphase Zeit eingespart wird.

Bei bevorzugten Ausführungsformen der erfindungsgemäßen Sicherheitsvorrichtung ist die Rückstellfeder am optischen Element oder an dessen Halter elektrisch leitend befestigt. Der Stromabgriff von dem verdrehbaren optischen Element bzw. Halter kann z.B. mittels eines Schleifers erfolgen. Bei bevorzugten Ausführungsformen der Erfindung ist jedoch das optische Element oder dessen Halter elektrisch leitend mit der Antriebsachse des Stellantriebs verbunden, an welcher der über die Rückstellfeder fließende Strom abgegriffen wird. Vorzugsweise wird dabei die Drehachse des optischen Elements durch die Antriebsachse des Stellantriebs gebildet. In einer Weiterbildung davon ist die Antriebsachse des Stellantriebs über ihr Drehlager leitend mit dem Gehäuse des Stellantriebs verbunden, an welchem dann der über die Rückstellfeder fließende Strom abgegriffen wird.

Weitere Vorteile der Erfindung ergeben sich aus der Beschreibung und der Zeichnung. Ebenso können die vorstehend genannten und die noch weiter aufgeführten Merkmale je für sich oder zu mehreren in beliebigen Kombinationen Verwendung finden. Die gezeigte und beschriebene Ausführungsform ist nicht als abschließende Aufzählung zu verstehen, sondern hat vielmehr beispielhaften Charakter für die Schilderung der Erfindung.

Es zeigen:
- Fig. 1: die Grundstellung der erfindungsgemäßen Sicherheitsvorrichtung für eine Laseranordnung;
- Fig. 2: die Betriebsstellung der in Fig. 1 gezeigten Sicherheitsvorrichtung; und
- Fig. 3: ein Blockschaltbild einer beispielhaften Überwachungsschaltung.

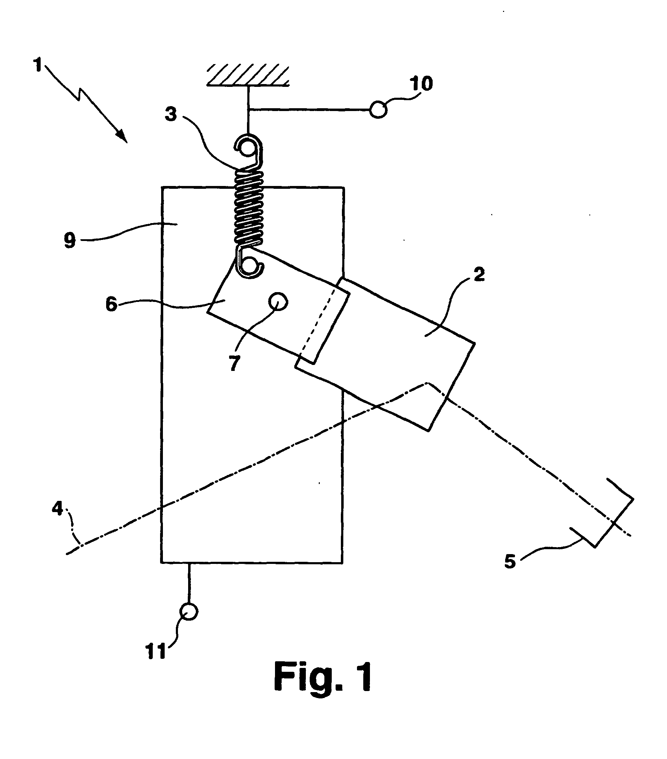

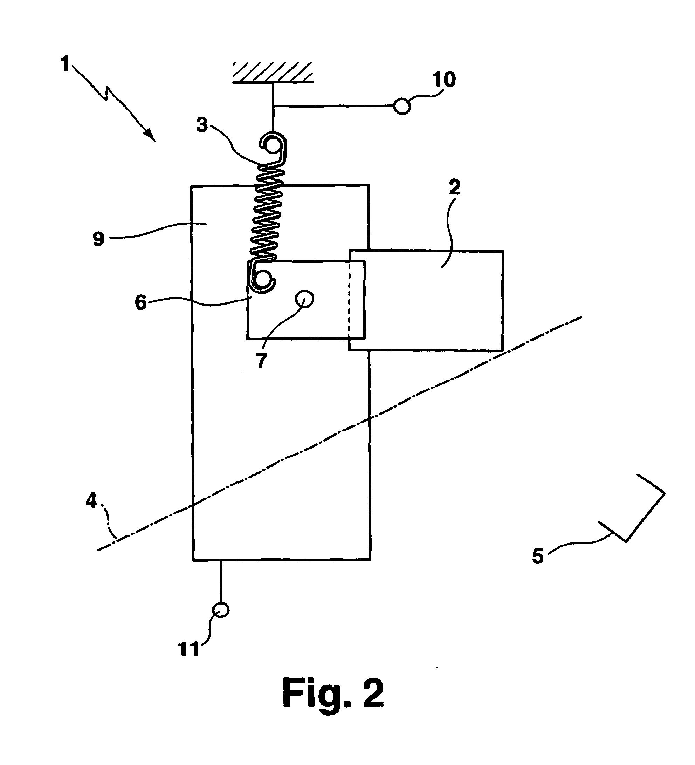

Die in **Fig. 1 und 2** gezeigte Sicherheitsvorrichtung **1** für eine Laseranordnung umfasst ein drehbar gelagertes optisches Element **2** , z.B. einen Spiegel, und einen Stellantrieb (nicht gezeigt), der das optische Element 2 gegen die Wirkung einer Rückstellfeder (Schraubenfeder) **3** aus der in Fig. 1 gezeigten Grundstellung in die in Fig. 2 gezeigte Betriebsstellung dreht. In seiner Grundstellung ist das optische Element 2 im Strahlengang eines Laserstrahls **4** angeordnet, der durch das optische Element 2 in einen Absorber **5** abgelenkt wird. Die Rückstellfeder 3 ist an einem Halter **6** des optischen Elements 2 exzentrisch zur Drehachse **7** befestigt, die durch die Antriebswelle des Stellantriebs gebildet ist.

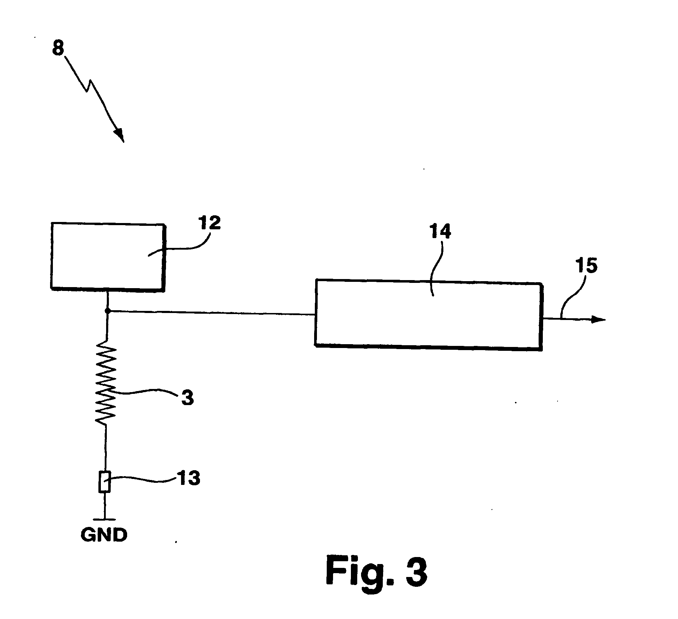

Die Rückstellfeder 3 ist im Stromkreis einer elektrischen Überwachungsschaltung **8** (Fig. 3) angeordnet, die eine Unterbrechung des über die Rückstellfeder 3 fließenden Stroms anzeigt. Dieser Strom wird an dem einen Ende der Rückstellfeder 3 und an dem Gehäuse **9** des Stellantriebs abgegriffen, welches über das Drehachsenlager (Kugellager), die Drehachse 7 und den Halter 6 elektrisch leitend mit dem anderen Ende der Rückstellfeder 3 verbunden ist. Die Kontakte zum Abgreifen des Stroms sind mit **10** und **11** bezeichnet.

Die in **Fig. 3** gezeigte Überwachungsschaltung 8 umfasst einen Sender **12**, der über die Rückstellfeder 3 und über Übergangswiderstände **13** mit Masse verbunden ist, sowie einen Empfänger **14** mit integrierter Auswertungseinheit. Der Sender 12 sendet fortlaufend Daten, z.B. ein vorbestimmtes Datenmuster, an den Empfänger 14. Ist die Datenübertragung gestört, wird in der Auswertungseinheit auf einen Bruch der Rückstellfeder 3 geschlossen und eine Fehlermeldung **15** ausgegeben.

## Patentansprüche

1. Sicherheitsvorrichtung (1) für eine Laseranordnung, mit einem drehbar gelagerten optischen Element (2), das in seiner Grundstellung im Strahlengang eines Laserstrahls (4) angeordnet ist, und mit einem Stellantrieb, der das optische Element (2) aus der Grundstellung gegen die Wirkung einer Rückstellfeder (3) in eine Betriebsstellung dreht, wobei die Rückstellfeder (3) am optischen Element (2) oder an dessen Halter (6) exzentrisch zur Drehachse (7) befestigt ist,
**dadurch gekennzeichnet,**
**dass** die Rückstellfeder (3) im Stromkreis einer elektrischen Überwachungsschaltung (8) angeordnet ist, welche eine Unterbrechung des über die Rückstellfeder (3) fließenden Stroms anzeigt.

2. Sicherheitsvorrichtung nach 1, **dadurch gekennzeichnet, dass** die Drehachse (7) des optischen Elements (2) durch die Antriebsachse des Stellantriebs gebildet ist.

3. Sicherheitsvorrichtung nach 1 oder 2, **dadurch gekennzeichnet, dass** die Rückstellfeder (3) am optischen Element (2) oder an dessen Halter (6) elektrisch leitend befestigt ist.

4. Sicherheitsvorrichtung nach 3, **dadurch gekennzeichnet, dass** das optische Element (2) oder dessen Halter (6) elektrisch leitend mit der Antriebsachse des Stellantriebs verbunden ist, an welcher der über die Rückstellfeder (3) fließende Strom abgegriffen wird.

5. Sicherheitsvorrichtung nach 4, **dadurch gekennzeichnet, dass** die Antriebsachse des Stellantriebs über ihr Drehlager elektrisch leitend mit dem Gehäuse (9) des Stellantriebs verbunden ist, an dem der über die Rückstellfeder (3) fließende Strom abgegriffen wird.

6. Sicherheitsvorrichtung nach einem der vorhergehenden Ansprüche, **dadurch gekennzeichnet, dass** der Laserstrahl (4) in der Grundstellung des optischen Elements (2) in einen Absorber (5) gelenkt ist.

## Claims

1. Safety device (1) for a laser arrangement, having a rotatably supported optical element (2) which is arranged in the beam path of a laser beam (4) in the basic position thereof, and having an actuating drive which rotates the optical element (2) out of the basic position into an operating position counter to the action of a restoring spring (3), the restoring spring (3) being secured to the optical element (2) or to the holder (6) thereof eccentrically relative to the axis of rotation (7),
**characterised in that**
the restoring spring (3) is arranged in the electrical circuit of an electrical monitoring circuit arrangement (8) which indicates an interruption of the electric current flowing via the restoring spring (3).

2. Safety device according to claim 1, **characterised in that** the axis of rotation (7) of the optical element (2) is formed by the drive shaft of the actuating drive.

3. Safety device according to claim 1 or 2, **characterised in that** the restoring spring (3) is secured in an electrically conductive manner to the optical element (2) or to the holder (6) thereof.

4. Safety device according to claim 3, **characterised in that** the optical element (2) or the holder (6) thereof is connected in an electrically conductive manner to the drive shaft of the actuating drive, at which drive shaft the electric current flowing via the restoring spring (3) is tapped.

5. Safety device according to claim 4, **characterised in that** the drive shaft of the actuating drive is connected in an electrically conductive manner via the rotary support thereof to the housing (9) of the actuating drive, at which housing (9) the electric current which flows via the restoring spring (3) is tapped.

6. Safety device according to any one of the preceding claims, **characterised in that** the laser beam (4) is directed into an absorber (5) when the optical element (2) is in the basic position.

## Revendications

1. Dispositif de sécurité (1) pour un système laser, avec un élément optique (2) monté à rotation, disposé, en une position de base, dans le trajet de cheminement des rayons laser (4), et avec un dispositif d'entraînement de réglage, faisant tourner l'élément optique (2) de la position de base, à l'encontre de l'effet d'un ressort de rappel (3), pour passer à une position de fonctionnement, le ressort de rappel (3) étant fixé sur l'élément optique (2) et/ou son support (6), de façon excentrique par rapport à l'axe de rotation (7),
**caractérisé en ce que**
le ressort de rappel (3) est disposé dans le circuit électrique d'un circuit de surveillance électrique (8) affichant une interruption du courant passant par le ressort de rappel (3).

2. Dispositif de sécurité selon la revendication 1, **caractérisé en ce que** l'axe de rotation (7) de l'élément optique (2) est formé par l'axe d'entraînement du dispositif d'entraînement de réglage.

3. Dispositif de sécurité selon la revendication 1 ou 2, **caractérisé en ce que** le ressort de rappel (3) est fixé de façon électriquement conductrice sur l'élément optique (2), ou sur son support (6).

4. Dispositif de sécurité selon la revendication 3, **caractérisé en ce que** l'élément optique (2), ou son support (6), est relié de façon électriquement conductrice à l'axe d'entraînement du dispositif d'entraînement de réglage, sur lequel est prélevé le courant passant par le ressort de rappel (3).

5. Dispositif de sécurité selon la revendication 4, **caractérisé en ce que** l'axe d'entraînement du dispositif d'entraînement de réglage est relié, par l'intermédiaire de son palier de rotation, de façon électriquement conductrice, au boîtier (2) du dispositif d'entraînement de réglage, sur lequel le courant, passant par le ressort de rappel (3), est prélevé.

6. Dispositif de sécurité selon l'une des revendications précédentes, **caractérisé en ce que** le rayon laser (4), à la position de base de l'élément optique (2), est dévié pour passer dans un absorbeur (5).
